# EUROPEAN PATENT SPECIFICATION

(11) **EP 3 043 770 B1**
(45) Date of publication and mention of the grant of the patent: **20.02.2019**
(21) Application number: 14790325.6
(22) Date of filing: 08.09.2014
(51) Int. Cl.: A61K 8/46, A61K 8/67, A61K 8/73, A61Q 17/04, A61Q 19/08

(54) **COSMETIC COMPOSITIONS CONTAINING A COMBINATION OF NATURAL ACTIVE INGREDIENTS**
KOSMETISCHE ZUSAMMENSETZUNGEN ENTHALTEND EINE KOMBINATION NATÜRLICHER AKTIVER INHALTSSTOFFE
COMPOSITIONS COSMÉTIQUES COMPRENANT UNE COMBINAISON D'ACTIFS NATURELS

(30) Priority: 09.09.2013 IT MI20131479
(43) Date of publication of application: 20.07.2016
(73) Proprietor: Iromed Group S.r.l., 00144 Roma (RM) (IT)
(72) Inventor: CARUSO, Ciro, I-80128 Napoli (NA) (IT); SANTANGELO, Rosaria, I-80016 Marano di Napoli (NA) (IT)
(74) Representative: Montelatici, Linda Anna
(86) International application number: PCT/IB2014/064314
(87) International publication number: WO 2015/033316

(56) References cited:
- EP-A1- 2 633 887
- WO-A2-2006/129149
- DE-A1-102004 002 602
- GB-A- 1 489 133
- US-A- 4 296 130
- US-A- 5 360 824
- US-A- 5 976 515
- US-A1- 2006 177 398
- DATABASE CA [Online] CHEMICAL ABSTRACTS SERVICE, COLUMBUS, OHIO, US; 26 April 2006 (2006-04-26), "De-scar and skin-nursing external health-care cream without side effect", XP002722267, Database accession no. 145:33491 & CN 1 762 325 A (ZOU FEI [CN]) 26 April 2006 (2006-04-26)
- DATABASE WPI Week 200943 Thomson Scientific, London, GB; AN 2009-K56387 XP002722268, "Preparation of anti-wrinkle beautifying cosmetics including facial cream, facial mask or skin milk comprises mixing acetyl hexapeptide-3 as anti-aging active agent and vitamin E with forming agents", & CN 101 450 027 A (NANJING RHINE PHARM TECHNOLOGY INC) 10 June 2009 (2009-06-10)
- DATABASE WPI Week 200864 Thomson Scientific, London, GB; AN 2008-K77599 XP002722269, "External preparation useful for treating dermatological disorders such as acne, keratosis, psoriasis, wrinkles and blotches, comprises adapalene and one or more of riboflavin, nicotinamide, panthenol, ascorbic acid or its salt and biotin", & JP 2008 184445 A (TAISHO PHARM CO LTD) 14 August 2008 (2008-08-14)

## Description

The present invention relates to novel cosmetic compositions for topical use on the skin and/or hair and/or nails, containing a combination of natural active principles.

Cosmetic creams comprising natural active principles, such as vitamins, are known.

For instance, creams and lotions for the skin containing riboflavin and/or vitamin E, are known.

Nevertheless, the known creams and lotions contain riboflavin and/or vitamin E only in traces, in particular riboflavin is contained in the known creams and/or lotions in amount generally well below 0.5% by weight.

CN1762325, DE102004002602, US5976515 disclose cosmetic compositions comprising riboflavin and vitamin E.

EP2633887 and CN20071191093 disclose cosmetic compositions comprising vitamin E.

US5360824 and JP20070020290 disclose cosmetic compositions comprising riboflavin.

US4296130 and US2006/177398 disclose cosmetic compositions comprising methylsulfonylmethane.

WO2006/129149 discloses poultry and livestock supplements that that comprise probiotics and methylsulfonylmethane.

Scope of the present invention is to provide a cosmetic composition for topical use on the skin and/or hair and/or nails, having excellent depigmenting, soothing, anti-wrinkle properties and comprising a combination of natural active ingredients. Such scope is achieved by a cosmetic composition whose main characteristics are defined in the first claim, while further characteristics are specified in the remaining claims.

According to the present invention, it was surprisingly found that a synergy of action exists in the combination of riboflavin and vitamin E in certain weight percentages, and that this synergistic effect is greatly increased if to this combination methylsulfonylmethane is added.

A first advantage consists therefore in that the cosmetic composition for topical use according to the invention containing methylsulfonylmethane has a soothing and anti-wrinkles effect which is much higher than that of compositions containing the same ingredients used singly and/or in different percentages.

Moreover, it was found that the combination of riboflavin and vitamin E in the above indicated percentages also has a protective effect against UVA radiations. This effect is further enhanced if vitamin E acetate is added. Therefore, a further advantage of the cosmetic compositions according to the invention consists in the great protective action against UVA radiations that is linked to a combination of natural active ingredients. The cosmetic compositions according to the invention can therefore be used to protect from the sun and they do not require additional sunscreens of chemical or physical type, such as benzophenone or benzoylmethane derivatives, aminobenzoic acid derivatives, salicylic acid or cinnamic acid derivatives, zinc oxide and titanium oxide, which are usually contained in the cosmetic compositions for protection from sunlight.

The cosmetic composition for topical use on the skin and/or hair and/or nails according to the present invention is in a form selected from ointment, cream, gel, paste and lotion, spreadable on the skin and/or hair and/or nails, and comprises a cosmetically acceptable excipient or carrier suitable for obtaining said composition in a form selected from ointment, cream, gel, paste and lotion, spreadable on the skin and/or hair and/or nails. Said composition comprises riboflavin and/or salts or hydrates thereof in a weight amount comprised between 0.05% and 0.50% by weight and vitamin E in a weight amount comprised between 0.10% and 2.00% and methylsulfonylmethane.

As the salt of riboflavin, the monosodium salt of riboflavin-5'-phosphate or the hydrate or dihydrate thereof, can be for instance used.

By vitamin E in the present description and in the claims is meant any one of the eight types of vitamin E or the tocotrienols (α, β, γ and δ) and the tocopherols (α, β, γ and δ), or a mixture thereof. Preferably, the composition according to the present invention comprises, as the vitamin E, alfa-tocopherol.

Preferably, in the cosmetic composition according to the present invention the amount of riboflavin and/or salts or hydrates thereof is comprised between 0.10% and 0.30% by weight, more preferably is comprised between 0.10% and 0.15% by weight, with respect to the total weight of the composition.

The amount of vitamin E, or of alfa-tocopherol, in the cosmetic composition according to the present invention, is preferably comprised between 0.30% and 1.50% by weight, more preferably said amount is comprised between 0.30% and 1.00%, with respect to the total weight of the composition.

The cosmetic composition for topical use on the skin and/or hair and/or nails according to the present invention comprises methylsulfonylmethane (MSM) as antioxidant agent.

The tests carried out by the inventors on a chicken chorioallantoic membrane, used as a toxicity model according to the international guidelines for non-invasive methods, have revealed the total absence of toxicity, both on a macroscopic scale and after microscopic analysis, of solutions containing riboflavin phosphate at a concentration of 0.125% (the same concentration, used in solutions for pharmaceutical use, such as, in particular, ophthalmic solutions for corneal cross-linking, did not show any kind of toxicity after the topical corneal application, even following the removal of the corneal epithelium). The results obtained reveal that no changes in the structure of the chorioallantoic membrane occur, and no alterations in the structure and number of the blood vessels close to the zone of application of the solutions containing riboflavin phosphate at 0.125%. By observation of the tissues in the vicinity of the zone of application with the scanning electron microscope, no morphological differences were revealed with respect to chicken chorioallantoic membranes not subjected to any treatments and used as negative control. The irradiation of the zone in question, with UV radiations in the ultraviolet absorption zone of type A (365 nm) revealed a great thickening of the chorioallantoic membrane in the presence of riboflavin phosphate only, whereas the use of supporting antioxidant, such as MSM and Coenzyme Q strongly reduced (approx. by half) the thickening observed.

Methylsulfonylmethane can be comprised in the cosmetic composition according to the present invention in a weight amount comprised between 0.05% and 2.0%, preferably between 0.10% and 2.00%; more preferably comprised between 0.30% and 1.50% by weight; even more preferably said amount is comprised between 0.30% and 1.00%, with respect to the total weight of the composition. Even more preferably, methylsulfonylmethane (MSM) is comprised in the cosmetic composition according to the present invention in a weight amount of 0.50%.

According to an aspect of the invention, the above described cosmetic composition has a strong protective effect from the UVA radiations and therefore it can be used for the protection of skin and hair from the sunlight.

According to another aspect, the cosmetic composition according to the invention is suitable for anti-wrinkles use and it may advantageously comprise also hyaluronic acid or a salt thereof.

Preferably, the weight amount of hyaluronic acid or of a salt thereof is comprised between 0.1% and 2.0%, more preferably between 0.1% and 1.0%, with respect to the total weight of the composition.

According to a further aspect of the invention, the cosmetic composition is indicated for the use in soothing.

The cosmetic compositions according to the present invention are in the form of ointments, creams, gels, pastes or lotions, and they can further comprise one or more additional substances selected from the group consisting of vitamins, humectants, emollients, soothing agents, antioxidants, chelating agents, preservatives, stabilizers, perfumes, emulsifiers, purified water.

As the vitamins, the composition according to the present invention can also comprise vitamin A, vitamin C, vitamins of the group B. Preferably, the composition according to the present invention comprises vitamin B5 or D-panthenol.

Advantageously, the composition according to the present invention comprises vitamin A acetate 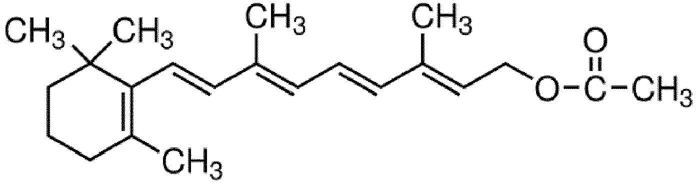 (formal name (2*E*,4*E*,6*E*,8*E*)-3,7-Dimethyl-9-(2,6,6-trimethylcyclohex-1-en-1-yl)nona-2,4,6,8-tetraen-1-yl acetate) in amount comprised between 6% and 25% by weight with respect to the total weight of the composition. Surprisingly, the Applicant have shown in experimental tests that the vitamin A acetate (also called retinyl acetate or retinol acetate) in such concentrations is a valid screen against ultraviolet rays, and helps to increase the sun protection factor (SPF) of the composition.

More generally, the Applicant has verified that the vitamin A acetate can be added to any cosmetic composition having a practically zero sun protection factor (SPF) in order to turn it into a cosmetic composition containing vitamin A acetate in amount comprised between 6% and 25% with respect to the total weight of the composition, having a sun protection factor (SPF) higher than 6 calculated according to EN ISO 24443:2012 issued in June 2012 by the European Standardisation Organisation (CEN). According to an embodiment, a cosmetic composition contains vitamin A acetate in amount lower than 23% with respect to the total weight of the composition.

More preferably, any cosmetic compositions containing vitamin A acetate in amount of at least 10% with respect to the total weight of the composition have a sun protection factor (SPF) which is considered not zero even according to the standard of 2012 adopted by the US FDA.

Contrary to what stated in the literature and commonly considered as true in the field, even as reported in the Material Safety Data Sheet of the compound, patch tests carried out by the Applicant have surprisingly shown that a cosmetic composition containing vitamin A acetate also in amount of 25% by weight with respect to the total weight of the composition does not cause rushes nor irritates the skin. This result is absolutely surprising because compounds that are relatively similar to the vitamin A acetate, such as retinol or retinyl palmitate, already in amount lower than 0.5% by weight with respect to the total weight of the composition, cause irritations to the skin and are typically poor tolerated by the most sensitive individuals.

As the humectants, vegetal glycerol, sorbitol, propylen glycol, butylen glycol, can be for instance used.

As emollient substances, can be used for instance octyl-2-dodecanol, olive oil, almond oil and their derivatives, essential fatty acids (vitamin F), caprylic/capric triglyceride, isononyl isonanoate, cetearyl alcohol, PEG-8 Stearate, ceteareth-25, butyrospermum parkii (Shea butter), glyceryl stearate, glyceryl resin, olea europaea unsaponifiable oil (olive), silicone oils, cyclopentasiloxane, cyclohexasiloxane, dimethicone.

As soothing agents, can be used for instance aloe vera, chamomille, allantoin, bisabolol, marigold, D panthenol.

As antioxidants, besides methylsulfonylmethane (MSM) can be also used for instance olive oil, almond oil and their derivatives, vitamin A, vitamin C, vitamins of group B.

As chelating agents, EDTA can be used for example.

As emollient cetyl PEG/PPG-10/1 dimethicone can be used.

The cosmetic composition according to the present invention can be prepared by a process comprising the following steps:
- preparation of a first water emulsion of riboflavin and/or salts or hydrates thereof and silicone oils, at a temperature comprised between 20°C and 40°C, preferably between 25°C and 30°C;
- preparation of a second water emulsion of vitamin E and optionally methylsulfonylmethane in water, at a temperature comprised between 70°C and 80°C, preferably at 75°±0.5°C, by emulsifying for a period of time not longer than 20 minutes, maintaining pH between 5 and 6;
- cooling the second emulsion to a temperature comprised between 20°C and 40°C, preferably between 25°C and 30°C;
- mixing the first emulsion and the second emulsion at a temperature comprised between 20°C and 40°C, preferably between 25°C and 30°C, and maintaining pH between 5 and 6.

The process according to the present invention can further comprise a step of addition of possible preservatives and/or perfumes.

According to an embodiment, a cosmetically acceptable carrier is an aqueous emulsion.

According to an embodiment, a cosmetic composition according to this description is liquid (for instance an emulsion oil in water), or creamy, or it is a gel.

The present description further provides a cosmetic method for preventing, ameliorating, reducing or treating the progressive degradation of the skin, for obtaining an improvement in the aesthetic appearance of the skin selected from the group consisting of: reduction of the dermatological signs of aging, photoaging, prevention and/or reduction of the appearance of wrinkles, reduction in the detectability of facial wrinkles, prevention and/or reduction of the depth of wrinkles, prevention and/or reduction of hyperpigmentation; increase of the skin elasticity, and any combination of them, the method comprising applying topically on the skin a cosmetic composition for topical use on the skin and/or hair and/or nails comprising riboflavin and/or salts or hydrates thereof in amount comprised between 0.05% and 0.50% by weight, vitamin E in amount comprised between 0.10% and 2.00% by weight with respect to the total weight of the composition, a cosmetically effective amount of methylsulfonylmethane and a cosmetically acceptable excipients or carrier suitable for making the composition spreadable on the skin and/or hair and/or nails. Preferably, the cosmetic method includes the application of the cosmetic cream at least once a day for two weeks; more preferably, twice a day (preferably in the morning and in the evening) for at least four weeks; even more preferably, twice a day (preferably in the morning and in the evening) for eight weeks.

The claims as filed are integral part of the description and they are incorporated herein by reference.

Further advantages and features of the compositions according to the present invention will be evident to any persons skilled in the art from the following detailed and non-limiting description of some embodiments.

The figure 1 is a graph of the average value of the cutaneous desquamation of the skin of volunteers observed every two weeks during the treatment with the cosmetic composition of example 2.

The figure 2 is a graph of the average value of cutaneous pigmentation of volunteers observed every two weeks during the treatment with the cosmetic composition of example 2.

The figure 3 is a graph of the average percentage value attributed to skin blemishes of volunteers observed every two weeks during the treatment with the cosmetic composition of example 2.

The figure 4 is a graph of the average values of the total elasticity (R2) and of the net elasticity (R5) of the skin of volunteers observed every two weeks during the treatment with the cosmetic composition of example 2.

The figure 5 is a graph of the average value attributed to the cutaneous isotropy of volunteers taken every two weeks during the treatment with the cosmetic composition of example 2.

The figure 6 is a graph of the average value attributed to the skin roughness of volunteers observed every two weeks during the treatment with the cosmetic composition of example 2.

### Example 1- Soothing cream

### Preparation of a phase A

In a turboemulsifier Jupiter 50/60 76,875 kg of water were weighed and the agitating blades were operated.

In the order were added, waiting for the complete solubilisation of each ingredient before adding the next one:
- 1 kg of vegetal glycerol;
- 1 kg of Aloe vera polysaccharides;
- 0,2 kg of chamomile water;
- 0,1 kg of EDTA;
- 0,125 kg of riboflavin 5 phosphate;
- 0,5 kg of MSM, paying attention to strictly maintain pH between 5 and 6;
- 0,5 kg of vitamin E TGPS.

At this time the heating jacket was operated up to a temperature of 75°C.

Then 0,3 kg of rheology modifier Tego® carbomer 140 were added, and the turboemulsifier was operated with a speed of 3000 rpm for about 8 minutes.

### Preparation of a phase B

In a fuser dissolver Antares having capacity of 200 1 at a temperature of 70°C were introduced:
4 kg of caprylic/capric triglycerides (Tegosoft® CT);
4 kg of isononyl isononanoate Acetamol In;
10 kg of Mixan 124S of the company Bregaglio S.r.l. (complex of emollient substances comprising cetearyl alcohol, PEG-8 Stearate, ceteareth-25, butyrospermum parkii (Shea butter), glyceryl stearate, glyceryl resin, olea europaea unsaponifiable oil);
1 kg of octyl-2-dodecanol.

The phases A and B were emulsified together in the turboemulsifier with a speed of 3000 rpm for about 20 minutes. Then, the emulsion was cooled down to about 25°C, under the action of the agitating blades, and then 0.1 kg of preservative Acnibio® Ac and 0,3 kg of perfume were added, always under agitation and finally controlling that the pH value is comprised between 5 and 6.

### Example 2- Anti-wrinkles cream

### Preparation of a phase A

In a turboemulsifier Jupiter 50/60 29,675 kg of water were weighed and the agitating blades were operated.

In the order were added, waiting for the complete solubilisation of each ingredient before adding the next one:
- 1 kg of Luviquat® PQ 11 PN;
- 1 kg of vegetal glycerol;
- 0,5 kg of D-panthenol;
- 0,2 kg of dry extract of melilotus;
- 0,1 kg of EDTA;
- 0,125 kg of riboflavin 5 phosphate;
- 0,5 kg of NaCl.

Preparation of a phase B: In a separated container were introduced 3 kg of a mixture of cyclopentylsiloxane and cyclohexasiloxane Abil® B 8839, 3 kg of dimethicone Abil® 350 and 4 kg of Cetyl PEG/PPG-10/1 Dimethicone Abil® Em 90.

Preparation of a phase C: The phases A and B were reunited under action of the turboemulsifier with a speed of 3000 rpm for 10 minutes, at the end the obtained product was poured into a steel container.

### Preparation of a phase D

In the turboemulsifier 40 kg of purified water were introduced and under agitation 0.5 kg of MSM were added, maintaining the pH value between 5 and 6.

Once the solubilisation was completed, 0.5 kg of vitamin E TGPS were added, and the heating jacket was operated up to a temperature of 75°C; finally 0.5 kg of sodium hyaluronate were added and the turboemulsifier was operated with a speed of 3000 rpm for about 6 minutes, until a viscous gel was obtained.

### Preparation of a phase E

In a fuser dissolver Antares having capacity of 200 1 at a temperature of 70°C were introduced:
5 kg of caprylic/capric triglycerides (Tegosoft® CT);
2 kg of isononyl isononanoate Acetamool In;
6 kg of Mixan 124S of the company Bregaglio S.r.l. (complex of emollient substances comprising cetearyl alcohol, PEG-8 Stearate, ceteareth-25, butyrospermum parkii (Shea butter), glyceryl stearate, glyceryl resin, olea europaea unsaponifiable oil);
1 kg of almond oil;
1 kg of olivene.

The phases D and E were hot emulsified together in the turboemulsifier with a speed of 3000 rpm for about 20 minutes. Then, the emulsion was cooled down to about 25°C, under action of the agitating blades, and 0.1 kg of preservative Acnibio® Ac and 0.3 kg of perfume were added, always under agitation and control that the pH value was comprised between 5 and 6.

The anti-wrinkles cream of example 2 was tested on a group of 20 volunteers between 40 and 70 years old who applied the cream twice a day for 8 weeks. At the beginning, during (every two weeks) and at the end of treatment in precisely determined zones of the face some parameters of the skin were measured, amongst these:
- cutaneous desquamation, sebum, pores by means of Visioscope PC35 (Courage & Khazaka) with the help of corneofix and sebufix;
- cutaneous elasticity by means of a Cutometer DUAL MPA 580 (Courage & Khazaka) with a standard probe (opening φ mm2) for the measurement of the cutaneous visco-elasticity for sucking. The skin surface was aspirated at the opening of the measuring probe, in which a constant level of vacuum (350 mbar) was created for a predetermined time (5 seconds). The depression was then brought to zero and the skin released to return to the original position. The software CK-MPA-Multi-Probe Adapter Cutometer Q & Courage Khazaka provided the value of the total elasticity (resistance to the ability to return) and of the net elasticity (elastic portion of the suction compared to the elastic portion of the relaxation);
- images of the wrinkles and numeric evaluation by a Visioscope PC35 (Courage & Khazaka);
- cutaneous thickness by using the Reviscometer RVM (Courage & Khazaka), an instrument for determining the cutaneous isotropy, able to measure the direction of the collagen and elastin fibres in order to determine the mechanical properties of the skin and of the skin aging;
- cutaneous colour by using a Mexameter MX 18 (Courage & Khazaka), able to measure the colour of the skin by means of the index of melanin and of erythema through their reflectance capacity;
- images of the cutaneous spots and numeric evaluation by means of Visioscope PC35 (Courage & Khazaka).

The measurements were carried out on the cheek, near the eye and on the forehead of the volunteer depending on the parameter to be measured in a reproducible manner.

The averages of each measurement were calculated at the beginning of treatment (T0), after two weeks (T1), after four weeks (T2), after six weeks (T3) and after eight weeks (Tf), obtaining the values shown in the figures from 1 to 6.

### Desquamation

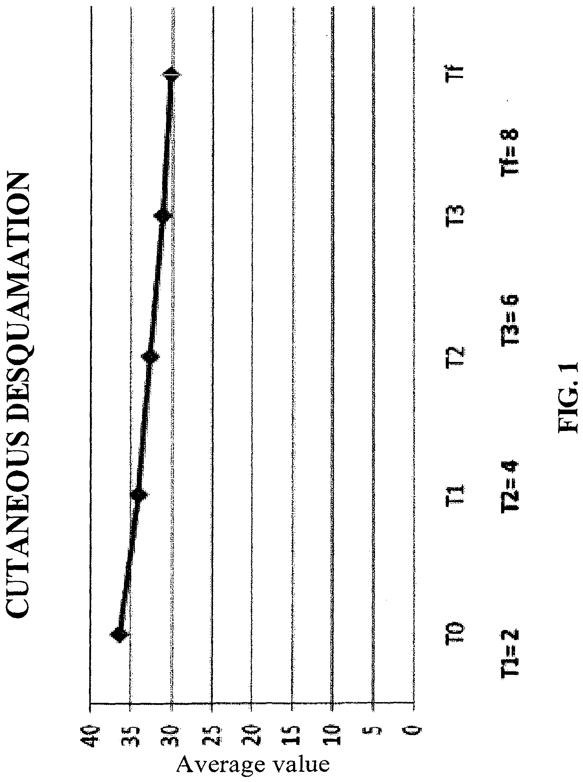

The desquamation values in all volunteers presented an average decrease of 17% in 8 weeks with a decreasing trend that was significant already starting from the second week of use (figure 1). It can be inferred that the decrease in the loss of the horny layer is correlated to a greater hydration and firmness of the skin hydrolipidic film in all subjects during the use of the cream.

### Depigmenting action

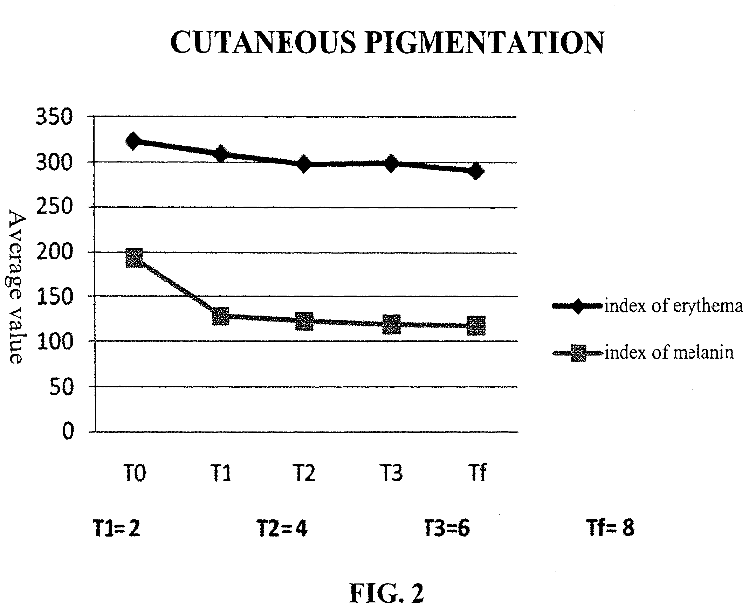

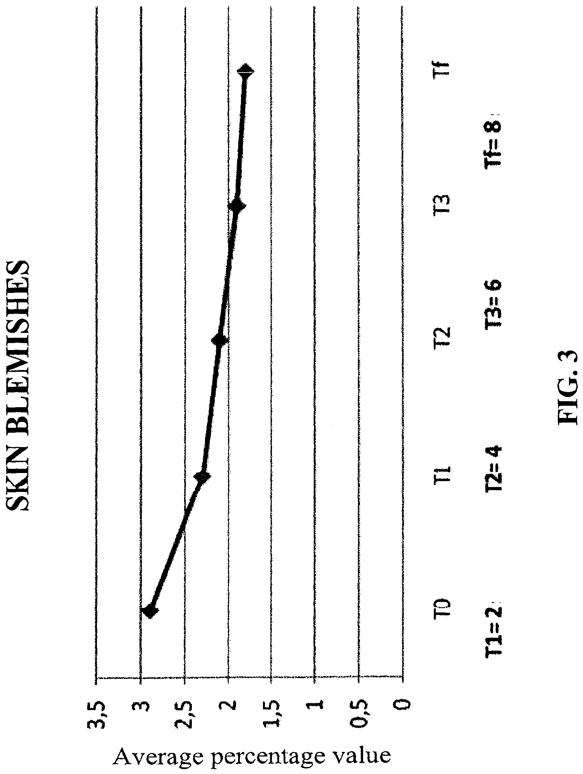

The index of erythema decreased significantly during the first month of treatment and then remained constant. The index of melanin decreased especially in the first two weeks of treatment and then remained constant in the subsequent period (figure 2). The pigmentation of skin spots was reduced steadily to decrease by 36% at the end of treatment (figure 3).

### Cutaneous elasticity and firming action

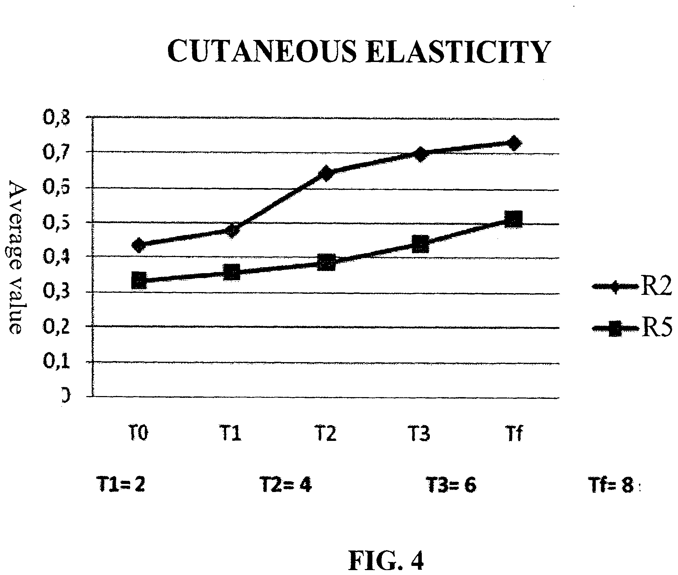

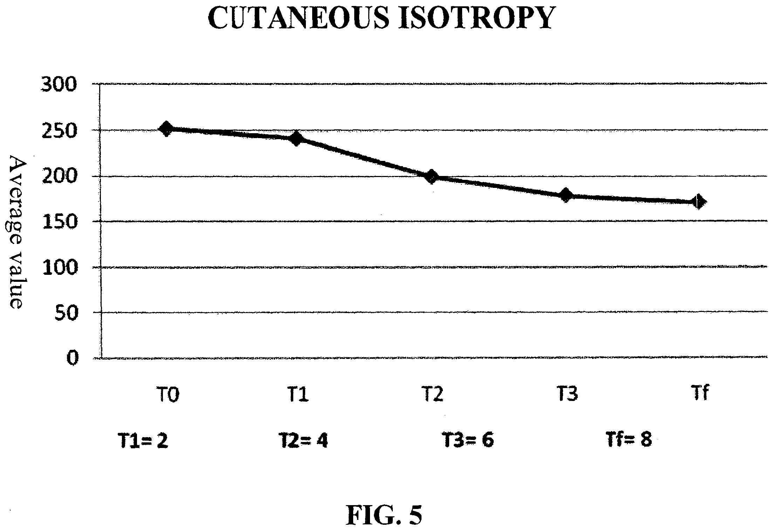

The total elasticity (parameter R2) increased during the whole period of use and resulted increased of 79% at the end of treatment. The net elasticity (parameter R5) increased during the whole period of use and resulted increased of 62% at the end of treatment (figure 4). The cutaneous isotropy diminished in a clear manner after 4 weeks and at the end of the 8 weeks of treatment achieved a 33% reduction (figure 5), that evidenced a firming effect of the skin of volunteers.

### Analysis of the wrinkles

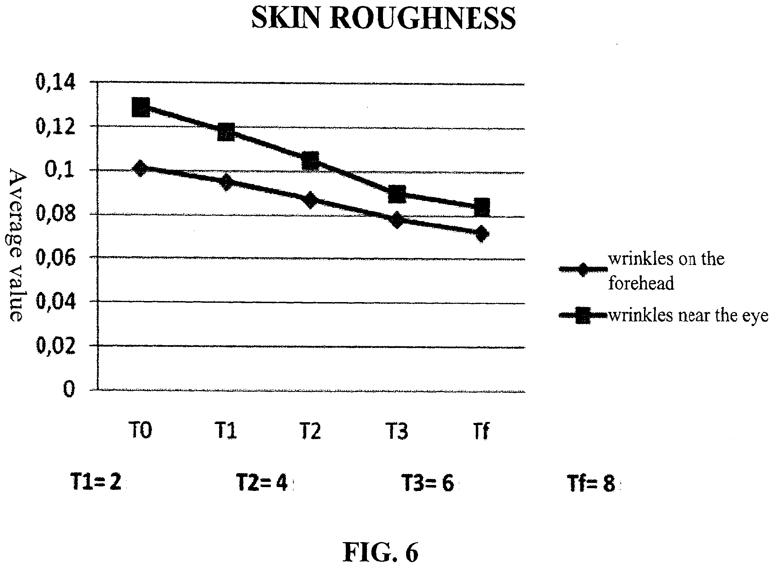

The image analysis and the related numerical parameters of the wrinkles near the eye and on the forehead of volunteers showed an average percentage decrease of 35% and 28% respectively at the end of treatment, with a decreasing trend substantially constant throughout the whole period of application (figure 6).

### Sebometry

The analysis of sebum on the cheek and the forehead and the amount of cutaneous pores on the cheek of volunteers showed a constant trend and in a normal range for almost all volunteers, so the cream did not increase the production of sebum. In those cases where the volunteer showed a high or scarce initial value of sebum, it went to normal values at the end of the eight weeks of application.

None of the volunteers showed any intolerance to the cream, or irritation or redness of the skin.

## Claims

1. A cosmetic composition for topical use on the skin and/or hair and/or nails, said composition being in a form selected from ointment, cream, gel, paste and lotion, spreadable on the skin and/or hair and/or nails, and comprising a cosmetically acceptable excipient or carrier suitable for obtaining said composition in a form selected from ointment, cream, gel, paste and lotion, spreadable on the skin and/or hair and/or nails, said composition being **characterised in that** it comprises riboflavin and/or salts or hydrates thereof in amount ranging between 0.05% and 0.50% by weight with respect to the total weight of the composition, vitamin E in amount ranging between 0.10% and 2.00% by weight with respect to the total weight of the composition, and methylsulfonylmethane.

2. The cosmetic composition according to the preceding claim, comprising methylsulfonylmethane in amount comprised between 0.10% and 2.00% by weight with respect to the total weight of the composition.

3. The cosmetic composition according to claim 1, wherein the amount of riboflavin and/or salts or hydrates thereof is comprised between 0.10% and 0.30% by weight with respect to the total weight of the composition.

4. The cosmetic composition according to claim 1, wherein the amount of vitamin E is comprised between 0.30% and 1.50 % by weight with respect to the total weight of the composition.

5. The cosmetic composition according to the preceding claims, comprising hyaluronic acid or a salt thereof in amount comprised between 0.10% and 2.00% by weight with respect to the total weight of the composition.

6. The cosmetic composition according to any one of the preceding claims, comprising one or more substances selected from the group consisting of vitamins, humectants, emollients, soothing agents, antioxidants, chelating agents, preservatives, stabilizers, and perfumes.

7. The cosmetic composition according to any one of the preceding claims, comprising vitamin A acetate in amount comprised between 6.00% and 25.00% by weight with respect to the total weight of the composition.

8. A process for manufacturing a cosmetic composition according to any one of the preceding claims, **characterized in that** said process comprises the steps of:
- preparing a first water emulsion of riboflavin and/or its salts or hydrates, and silicone oils, at a temperature comprised between 20°C and 40°C;
- preparing a second water emulsion comprising vitamin E and methylsulfonylmethane, at a temperature comprised between 70°C and 80°C, by emulsifying for a period not exceeding 20 minutes, maintaining pH between 5 and 6;
- cooling said second emulsion to a temperature of between 20°C and 40°C;
- mixing said first emulsion with said second emulsion at a temperature of between 20°C and 40°C, maintaining pH between 5 and 6.

9. The process according to the previous claim, wherein the step of preparing said second emulsion is carried out at 75 ± 0.5 ° C.

## Patentansprüche

1. Eine kosmetische Zusammensetzung zur örtlichen Anwendung auf der Haut und/oder an den Haaren und/oder Nägeln, wobei die besagte Zusammensetzung eine Form aufweist, ausgewählt aus Salbe, Creme, Gel, Paste und Lotion, verteilbar auf der Haut und/oder den Haaren und/oder Nägeln, und umfassend einen kosmetisch akzeptablen Hilfsstoff oder Träger, der dafür geeignet ist, die besagte Zusammensetzung in einer Form ausgewählt aus Salbe, Creme, Gel, Paste und Lotion, die auf der Haut und/oder den Haaren und/oder Nägeln verteilbar ist, zu enthalten, wobei die besagte Zusammensetzung **dadurch gekennzeichnet ist, dass** sie Riboflavin und/oder Salze oder Hydrate davon in einer Menge zwischen 0,05 Gew.-% und 0,50 Gew.-% umfasst, bezogen auf das Gesamtgewicht der Zusammensetzung, Vitamin E in einer Menge zwischen 0,10 Gew.-% und 2,00 Gew.-%, bezogen auf das Gesamtgewicht der Zusammensetzung, sowie Methylsulfonylmethan.

2. Die kosmetische Zusammensetzung gemäß dem vorangehenden Anspruch, umfassend Methylsulfonylmethan in einer Menge zwischen 0,10 Gew.-% und 2,00 Gew.-%, bezogen auf das Gesamtgewicht der Zusammensetzung.

3. Die kosmetische Zusammensetzung gemäß Anspruch 1, wobei die Menge des Riboflavins und/oder von Salzen oder Hydraten davon zwischen 0,10 Gew.-% und 0,30 Gew.-% liegt, bezogen auf das Gesamtgewicht der Zusammensetzung.

4. Die kosmetische Zusammensetzung gemäß Anspruch 1, wobei die Menge des Vitamin E zwischen 0,30 Gew.-% und 1,50 Gew.-% liegt, bezogen auf das Gesamtgewicht der Zusammensetzung.

5. Die kosmetische Zusammensetzung gemäß den vorhergehenden Ansprüchen, umfassend Hyaluronsäure oder ein Salz davon in einer Menge zwischen 0,10 Gew.-% und 2,00 Gew.-%, bezogen auf das Gesamtgewicht der Zusammensetzung.

6. Die kosmetische Zusammensetzung gemäß einem der vorhergehenden Ansprüche, umfassend eine oder mehrere Substanzen, ausgewählt aus der Gruppe bestehend aus Vitaminen, Befeuchtungsmitteln, Weichmachern, Beruhigungsmitteln, Antioxidantien, Chelatbildnern, Konservierungsmitteln, Stabilisatoren und Duftstoffen.

7. Die kosmetische Zusammensetzung gemäß einem der vorhergehenden Ansprüche, umfassend Vitamin-A-Acetat in einer Menge zwischen 6,00 Gew.-% und 25,00 Gew.-%, bezogen auf das Gesamtgewicht der Zusammensetzung.

8. Ein Verfahren zur Herstellung einer kosmetischen Zusammensetzung nach einem der vorhergehenden Ansprüche, **dadurch gekennzeichnet, dass** der besagte Prozess die folgenen Schritte umfasst:
- Zubereiten einer ersten wässrigen Emulsion von Riboflavin und/oder seiner Salze oder Hydrate, und von Silikonölen bei einer Temperatur zwischen 20 °C und 40 °C;
- Zubereiten einer zweiten wässrigen Emulsion, umfassend Vitamin E und Methylsulfonylmethan, bei einer Temperatur zwischen 70 °C und 80 °C, durch Emulgieren für eine Dauer von nicht mehr als 20 Minuten, unter Einhaltung eines pH-Wertes zwischen 5 und 6;
- Abkühlen der besagten zweiten Emulsion auf eine Temperatur zwischen 20 °C und 40 °C;
- Vermischen der besagten ersten Emulsion mit der besagten zweiten Emulsion bei einer Temperatur zwischen 20 °C und 40 °C, unter Einhaltung eines pH-Wertes zwischen 5 und 6.

9. Das Verfahren gemäß dem vorangehenden Anspruch, wobei der Schritt des Vorbereitens der besagten zweiten Emulsion bei 75 ± 0,5 °C ausgeführt wird.

## Revendications

1. Composition cosmétique à usage topique à appliquer sur la peau et/ou les cheveux et/ou les ongles, ladite composition étant sous une forme choisie parmi la pommade, la crème, le gel, la pâte et la lotion, pouvant s'étaler sur la peau et/ou les cheveux et/ou les ongles, et comprenant un excipient ou véhicule acceptable sur le plan cosmétique permettant d'obtenir ladite composition sous une forme choisie parmi la pommade, la crème, le gel, la pâte et la lotion, pouvant s'étaler sur la peau et/ou les cheveux et/ou les ongles, ladite composition étant **caractérisée en ce qu'**elle comprend de la riboflavine et/ou un de ses sels ou hydrates en une quantité dans la plage de 0,05 à 0,50 % en poids par rapport au poids total de la composition, de la vitamine E en une quantité dans la plage de 0,10 à 2,00 % en poids par rapport au poids total de la composition, et du méthylsulfonylméthane.

2. Composition cosmétique selon la revendication précédente, comprenant du méthylsulfonylméthane en une quantité comprise entre 0,10 et 2,00 % en poids sur la base du poids total de la composition.

3. Composition cosmétique selon la revendication 1, dans laquelle la quantité de riboflavine et/ou de ses sels ou hydrates est comprise entre 0,10 et 0,30 % en poids sur la base du poids total de la composition.

4. Composition cosmétique selon la revendication 1, dans laquelle la quantité de vitamine E est comprise entre 0,30 et 1,50 % en poids sur la base du poids total de la composition.

5. Composition cosmétique selon les revendications précédentes, comprenant de l'acide hyaluronique ou un de ses sels en une quantité comprise entre 0,10 et 2,00 % en poids sur la base du poids total de la composition.

6. Composition cosmétique selon l'une quelconque des revendications précédentes, comprenant une ou plusieurs substances choisies dans le groupe constitué par les vitamines, les agents hydratants, les émollients, les adoucissants, les antioxydants, les agents chélateurs, les conservateurs, les stabilisants et les parfums.

7. Composition cosmétique selon l'une quelconque des revendications précédentes, comprenant de l'acétate de vitamine A en une quantité comprise entre 6,00 et 25,00 % en poids sur la base du poids total de la composition.

8. Procédé de préparation d'une composition cosmétique selon l'une quelconque des revendications précédentes, **caractérisé en ce que** ledit procédé comprenant les étapes suivantes :
- préparation d'une première émulsion aqueuse de riboflavine et/ou de ses sels ou hydrates, et d'huiles silicone, à une température comprise entre 20 et 40°C;
- préparation d'une seconde émulsion aqueuse comprenant la vitamine E et le méthylsulfonylméthane, à une température comprise entre 70 et 80 °C, par émulsification pendant une durée n'excédant pas 20 minutes, et maintien du pH entre 5 et 6 ;
- refroidissement de ladite seconde émulsion à une température entre 20 et 40°C;
- mélange de ladite première émulsion avec ladite seconde émulsion à une température entre 20 et 40 °C, et maintien du pH entre 5 et 6.

9. Procédé selon la revendication précédente, dans lequel l'étape de préparation de ladite seconde émulsion est effectuée à 75 ± 0,5 °C.
